# EUROPEAN PATENT SPECIFICATION

(11) **EP 2 926 127 B1**
(45) Date of publication and mention of the grant of the patent: **06.05.2020**
(21) Application number: 13792200.1
(22) Date of filing: 05.11.2013
(51) Int. Cl.: G01N 31/22, A61L 2/28, G01K 3/04, G01K 11/06, A61L 2/07

(54) **AIR REMOVAL TEST STRIP**
TESTSTREIFEN FÜR ENTFERNUNG VON LUFT
BANDELETTE DE TEST POUR ÉLIMINER AIR

(30) Priority: 28.11.2012 US 201213687888
(43) Date of publication of application: 07.10.2015
(73) Proprietor: American Sterilizer Company, Mentor, OH 44060 (US)
(72) Inventor: BALA, Harry, South Barrington, IL 60010 (US)
(74) Representative: Somervell, Thomas Richard
(86) International application number: PCT/US2013/068464
(87) International publication number: WO 2014/085038

(56) References cited:
- EP-A1- 0 022 284
- WO-A1-95/24622
- US-A- 4 410 493
- US-A- 5 378 430
- US-A- 5 895 627
- US-A1- 2009 029 451

## Description

### BACKGROUND OF THE INVENTION

It is well known that heat destroys microorganisms. The presence of moisture accelerates this destruction by denaturing or coagulation of the proteins making up the microorganisms. Most microorganisms contain sufficient water so that moderate heat alone, e.g. 80°C-100°C, will destroy the microorganism. Many bacterial spores, on the other hand, contain substantially no water and require elevated temperatures in excess of 150°C for their destruction where dry heat is used. Hence, the destruction of such organisms is generally carried out in the presence of steam in steam sterilizers.

There are two basic types of steam sterilizers (autoclaves) - a gravity displacement autoclave and a prevacuum type sterilizer. In the gravity displacement autoclaves, steam is admitted at the top or the sides of the sterilizing chamber and, because the steam is lighter than air, forces air out the bottom of the chamber through a drain vent. Gravity displacement autoclaves are primarily used to process laboratory media, water, pharmaceutical products, regulated medical waste, and nonporous articles whose surfaces have direct steam contact. For gravity displacement sterilizers the penetration time into porous items is prolonged because of incomplete air elimination. For example, decontamination of 10 lbs of microbiological waste requires at least 45 minutes at 121°C, because the entrapped air remaining in a load of waste greatly retards steam permeation and heating efficiency.

Prevacuum type sterilizers are similar to the gravity displacement sterilizers except they are fitted with a vacuum pump (or ejector) to ensure air removal from the sterilizing chamber and load before the steam is admitted. By removing air, nearly instantaneous steam penetration can be provided even into porous loads.

Bowie-Dick type air removal tests are used to detect air leaks and inadequate air removal in prevacuum sterilizers. In the Bowie-Dick test, a commercially available Bowie-Dick-type test indicator is placed in the center of a test pack consisting of folded 100% cotton surgical towels prepared according to a standard test method, such as American National Standards Institute (ANSI)/Association for the Advancement of Medical Instrumentation (AAMI)/International Organization for Standards (ISO) 11140-5:2007. The test pack is placed horizontally in the front, bottom section of a sterilizer rack, near the door and over the drain, in an otherwise empty chamber, and the sterilizer is tested. Successful air removal is verified by a uniform color change in the Bowie-Dick-type test indicator after exposure to saturated steam at 134°C for 3.5min ± 5 sec and/or at 121°C for 15min ± 5 sec.

Inadequate air removal can result in an inadequate sterilization process, since air that is not removed from the chamber can interfere with steam contact. Thus, the air removal test is performed each day the vacuum-type steam sterilizer is used, before the first processed load. The test packs can be prepared using freshly laundered cotton surgical towels. ANSI/AAMI/ISO 11140-5:2007 requires that the towels to be folded to a size 250 mm ± 20mm in one direction and 300 mm ± 20mm in the other direction, and that the height of the test pack to be between 250 mm and 280 mm. Thus, preparation for the air removal test for users can be labor intensive, time consuming and cost prohibitive. Disposable air removal test systems including disposable test packs have been developed, but they are still costly and create waste from disposing test packs after each use.

Therefore, there is a need for an improved air removal test system that can provide indication for successful air removal in prevacuum type sterilizers.

### BRIEF SUMMARY OF THE INVENTION

In one aspect, a test strip for verifying adequate air removal during a sterilization cycle of a pre-vacuum type sterilizer is provided. The test strip includes a base element, a solid indicator chemical, a wicking material, a film and a coated paper. The base element is formed from a thermally conductive material having a length and a width, and having a recess formed therein. The solid indicator chemical is deposited in the recess. The wicking material is positioned at least in part in contact with the solid indicator chemical, and extends less than the length and width of the base element. The film is positioned over the base element, the wicking material, and the solid indicator chemical. The coated paper is disposed over the film, and includes a window. The widow is arranged away from the solid indicator chemical such that a portion of the wicking material is visible through the window. The test strip is configured such the solid indicator chemical liquefies and wicks along the wicking material during a pre-vacuum sterilization cycle and changes a color of the wicking material. Further, the test strip is configured to verify adequate air removal when the color change covers the portion of the wicking material visible through the window.

The air removal test system of the present invention is defined in claim 1.

In another aspect, an air removal test system for verifying adequate air removal during a sterilization cycle of a pre-vacuum type sterilizer is provided. The test system includes a specimen holder and the above described test strip. The specimen holder includes a tubular body defining an interior chamber, a closure cap sealable on a first end of the tubular body, and an end plug arranged in a second end of the tubular body. The end plug provides a single ingress and egress flow path into and out of the interior chamber. The test strip is placed in the interior chamber of the specimen holder, which is then placed in a pre-vacuum type sterilizer. In the sterilizer, the solid indicator chemical liquefies and wicks along the wicking material during a pre-vacuum sterilization cycle and changes a color of the wicking material. The test strip is configured to verify adequate air removal when the color change covers the portion of the wicking material visible through the window.

In one embodiment, the base element has an adhesive backing, for example, an acrylic adhesive. The solid indicator chemical is salicylamide, ethoxy benzomide or similar chemical, and can also include a dye. For example, the solid indicator chemical can include blue color dye present in a concentration of about 0.01 percent by weight of the indicator chemical. Further, the film is a cast polypropylene having a thickness of about 2.0 to 2.2 mils or about 3.0 to 3.2 mils
1 mil corresponds to 25,4 micrometers. The base element can be aluminum having a thickness of about 3 mils. Further, the coated paper can have an adhesive backing and an acrylic coating thereon.

These and other features and advantages of the present invention will be apparent from the following detailed description, in conjunction with the appended claims.

### BRIEF DESCRIPTION OF THE SEVERAL VIEWS OF THE DRAWINGS

The benefits and advantages of the present invention will become more readily apparent to those of ordinary skill in the relevant art after reviewing the following detailed description and accompanying drawings, wherein:
FIG. 1 is a top plan view of an air removal test strip according to an embodiment;
FIG. 2 is a cross-sectional view taken along line 2--2 of FIG. 1;
FIG. 3 is a top perspective view of the test strip of FIG. 1 with a paper layer, an adhesive layer, and a film layer peeled off;
FIG. 4 is an exploded view of a specimen holder according to an embodiment; and
FIG. 5 is a cross-sectional view of the specimen holder of FIG. 4.

### DETAILED DESCRIPTION OF THE INVENTION

While the present invention is susceptible of embodiment in various forms, there is shown in the drawings and will hereinafter be described a presently preferred embodiment with the understanding that the present disclosure is to be considered an exemplification of the invention and is not intended to limit the invention to the specific embodiment illustrated.

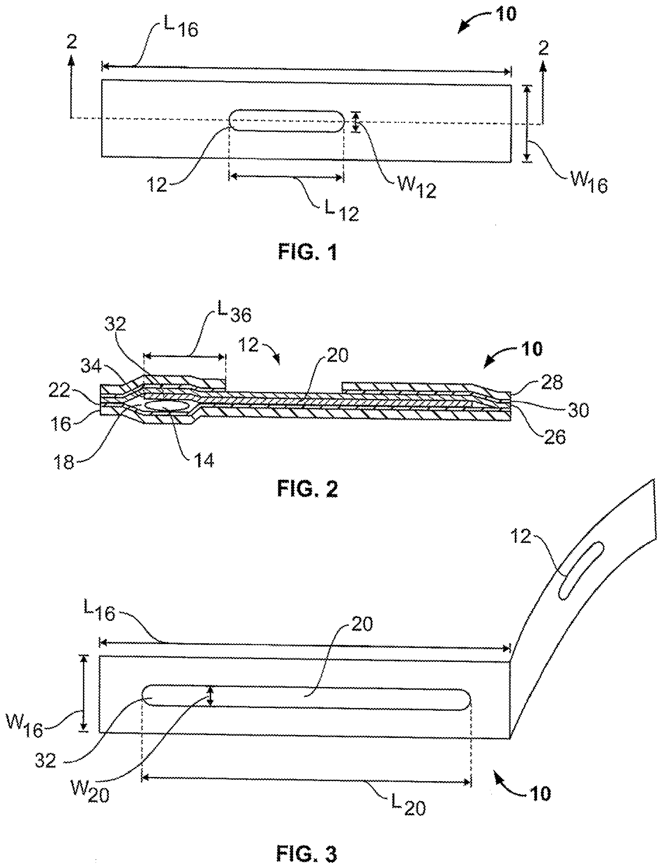

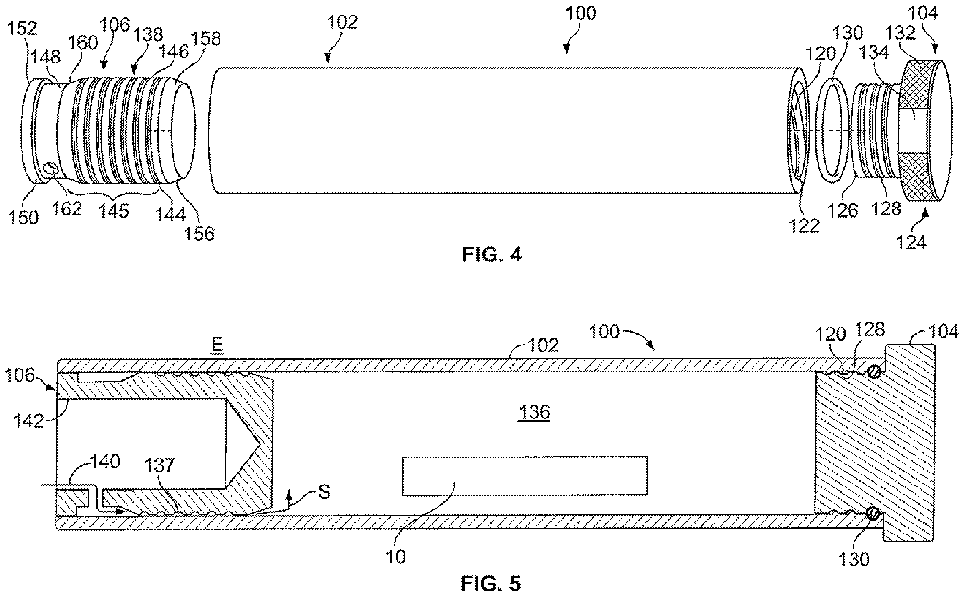

FIGS. 1-3 show an embodiment of a test strip 10 for detecting air leaks and inadequate air removal in a pre-vacuum type sterilizer. The strip 10 is configured to work with a reusable specimen holder, such as those disclosed in US 7,718,125, US 7,740,802, US 7,790,105, and US 7,811,516, which are commonly assigned to the assignee of the present application. A specimen holder 100 according to an embodiment is shown in FIGS. 4-5. In use, the test strip 10 is placed in the specimen holder 100 as shown in FIG. 5, which is then placed in a pre-vacuum type sterilizer for a pre-vacuum sterilization cycle to determine whether an adequate air removal in the sterilization has been achieved.

As shown in FIG. 1, the strip 10 has an open window 12 through which the wicking of an indicator chemical 14 (FIG. 2) can be observed to determine whether adequate air removal in the prevacuum has occurred as will be described below. FIG. 2 is a cross-sectional illustration of the strip 10. The strip 10 generally includes a base element 16, an adhesive layer 22, a wicking element 20, a film layer 26, an adhesive layer 30, a paper layer 28, and a solid indicator chemical 14. The base element 16 is formed from a foil or other high-heat transfer material. The adhesive layer 22 is provided on the base element 16 as a continuous layer covering substantially the entire top surface of the base element 16. The base element 16 has a length L₁₆ and a width W₁₆. In this embodiment, the base element 16 is formed from an aluminum foil. A depression or recess 18 is formed in the base element 16 and the adhesive layer 22. The indicator chemical 14, which is a temperature sensitive chemical formulation, is provided in the recess 18 between the adhesive layer 22 and the wicking element 20.

The wicking element 20, such as a wicking paper is positioned on the base element 20 attached by the adhesive layer 22. FIG. 3 shows a top view of the strip with the film layer 26, the adhesive layer 30, and the paper layer 28 peeled away to illustrate the wicking element 20 arranged on the base element 20 and the adhesive layer 22. As shown, the wicking element 20 has a width W₂₀ and a length L₂₀ that is less than the width W₁₆ and the length L₁₆ of the base element 16. The wicking element 20 is generally centered on the base element 16 and over the indicating chemical 14 in the recess 18, such that an end portion 32 of the wicking element 20 is in contact with the indicator chemical 14. The wicking element 20 extends longitudinally along the base element 16, such that at least some portion of the wicking element is securely attached to the base element 20 by the adhesive layer 22. In this manner, the indicator chemical 14 and the wicking element 20 are bounded within the four sides of the test strip 10.

The film 26 is applied over the wicking element 20, and adhered to the base element 16 by the adhesive layer 22. The film 26 has substantially the same width and length as the base element 16, and thus, it is attached to the base element 16 by the adhesive layer 22 in the peripheral areas around the wicking element 20. The film 26 is a transparent film, as will be discussed in more detail below. The paper layer 28 is provided with the adhesive layer 30, and applied over the film 26. The paper layer 28 and the adhesive layer 30 include the window 12 that is cut out (as seen in FIGS. 1-3) to allow for visual indication within the window 12, through the film 26.

In exemplary test strips 10, the foil element 16 and the adhesive layer 22 are formed using a 3/1000 inch (3 mil) thick adhesive label. The adhesive layer 22 on the foil 16 (to adhere to the wicking element 20 and the film 26) is an acrylic adhesive. The paper layer 28 and the adhesive layer 30 are formed from an acrylic coated paper.

The film layer 26 is formed from a cast polypropylene having a thickness of about 2.0 to 2.2 mils or 3.0 to 3.2 mils. The wicking element 20 is formed from a suitable wicking material, for example, a wicking material commercially available from Whatman Inc. of Piscataway, New Jersey under the product identifier Whatman 1. This wicking material is a low-ash, qualitative paper having a basis weight of about 66 grams per square meter (g/m²) and a caliper or thickness of about 7.3 thousandths of an inch (mils).

The indicator chemical 14 is salicylamide, ethoxy benzomide or a similar chemical, having a colorant added in a concentration of about 0.01 percent by weight. An example of one colorant is a blue color dye.

The window 12, which is formed in the paper layer 28 and the adhesive layer 30, extends over an area of the surface of the paper layer 28 less than the entirety of the length of the paper 28. Further, the window 12 has a length L₁₂ and a width W₁₂, which are less than the length L₂₀ and the width W₂₀ of the wicking element 20. The window 12 is arranged over the wicking element 20, such that only a portion of the wicking element 20 is visible through the film layer 26 and the window 12.

The test strip 10 is configured such that when the test strip 10 is inserted in the specimen holder 100 and placed in a pre-vacuum type sterilizer chamber, the test strip 10 can indicate whether adequate air removal has occurred during a sterilization cycle, which provides saturated steam at 134°C for 3.5min ± 5 sec and/or at 121°C for 15min ± 5 sec, as does a Bowie-Dick-type test indicator system prepared according to ANSI/AAMI/ISO 11140-5:2007. During a sterilization cycle, the indicator chemical 14 liquefies and wicks along the wicking material 20. In preferred embodiments, adequate air removal is verified when the liquefied indicator chemical wicks passed the window 12. That is, the color of the liquefied indicator chemical, for example, blue, must cover substantially the entire portion of the wicking material 20 visible through the window 12 to indicate that air has been adequately removed from the sterilizer chamber during a pre-vacuum sterilizing cycle. Therefore, the window 12 is sized and arranged at a precalculated distance from the location of the indicator chemical 14 according to particular pre-vacuum steam sterilization parameters.

In one embodiment, the test strip 10 has a length L₁₆ of about 4 inches and a width W₁₆ of about 3/4 inches. The wicking element 20 has a length L₂₀ of about 3 3/8 inches and a width W₁₆ of about 1/4 inches, and arranged generally in the middle of the base element 16 as shown in FIG. 3. The indicator chemical 14 is placed under the end portion 32 of the wicking element 20 as shown in FIG. 2. The window 12 has a length L₁₂ of about 1 inches and a width W₁₂ of about 3/16 inches, and is arranged longitudinally away from the indicator chemical 14. In this embodiment, the window 12 is positioned away from an end 34 of the wicking element 20 a distance L₃₆ of about 15/16 inches. The window 12 is arranged over the wicking element 20, such that the wicking of the liquefied indicator chemical 14 can be observed through the window 12. In this embodiment, adequate air removal during a steam sterilization cycle is verified when the color change or a dark bar formed from the wicking of the liquefied indicator chemical covers substantially the entire area of the wicking element 20 visible through the window 12.

Various embodiments of the strip 10 can be configured to work with reusable specimen holders, such as sterilization challenge specimen holders disclosed in US 7,718,125, US 7,740,802, US 7,790,105, and US 7,811,516. An exemplary specimen holder 100 is shown in FIGS. 4-5. The holder 100 includes a hollow tubular body 102, a closure cap 104 and an end plug 106. The body 102 can be covered or enveloped in an insulating layer (not shown.)

The body 102 includes an internal thread 120 at one open end 122. The closure cap 104 includes a gripping portion 124 and a depending plug 126 having an external thread 128 (to mate to the threaded 120 opening) to close the holder 100. A seal 130 such as the illustrated O-ring can fitted onto the cap 104 to provide a gas-tight seal between the closure cap 104 and the body 102. In a present holder 100, the gripping portion 124 is textured or knurled (as indicated at 132) to facilitate rotating or turning the cap 104. The gripping portion 124 can include a flattened portion (a flat 134) so that when the holder 100 is laid on its side, it will be prevented from rolling.

The end plug 106 provides a single ingress and egress flow path into and out of the interior or chamber 136 when the cap 104 is in place on the body 102 and permits drawing a vacuum in the holder 100 and introducing a sterilization fluid, such as steam, into to the holder 100 in a controlled manner. The plug 106 includes a body 138 having a recess or well 140 formed therein that defines an inner wall 142. The body 138 includes a spiral formed channel or groove 144 in an outer wall 146 thereof. The outer wall 146 includes a peripheral recess or channel 148 formed adjacent to a sealing lip 150 at an end 152 of the plug 106.

The spiral formed channel 144 opens at a first end 152 into the peripheral recess 148 and spirals around the body 138 extending to about the opposite end 156 of the plug 106. In a present plug 106, the opposite end 156 (which is the end at the chamber 136 side of the plug 106), includes a chamfer 158 at which the spiral formed groove 144 ends. In a present plug 106, the transition from the peripheral channel 148 to the grooved region 145 is also chamfered as indicated at 160.

The plug 106 includes an opening 162 through the wall 146 at the peripheral recess 148. The opening 162 provides communication between outside of the holder 100 (the environs E) and the interior or chamber 136 of the holder 100. Communication is provided from the environs E, through the opening 162, into the recess 148, through the spiral groove144 and into the chamber 136. The chamfers 158, 160 at both ends of the spiral groove 144 (or grooved region 145) provide for a smooth transition into and out of the groove 144 and prevent excessive resistance to flow through the groove 144. The groove 144, is smooth, as by being formed by machining, but provides a tortuous ingress and egress path between the environs E and the chamber 136.

The plug 106 is friction fitted into the chamber body 102. In this manner the lip 150 is snug up to the interior wall 137 of the body 102 and provides an external seal between the environs E and the peripheral recess 148. Moreover the plug body outer wall 146, at the grooved region 145 (between the chamfers 158, 160) also is snug up to the interior wall 137 of the body 102 and provides a seal between the recess 148 and the chamber 136 and the groove 144. It will be appreciated that the interior wall 137 of the body, at the plug 106 is smooth, unlike the threads 120 formed in the cap 104 end. In that there is no machining necessary at interior wall 137 the smooth surface (unlike threads) proper "mating" of the plug 106 and body 102 is enhanced.

A present holder 100 is formed from aluminum. Many different materials are contemplated for use, including various other metals, steels, alloys and the like. Suitable polymers may also be used, as will be appreciated by those skilled in the art. Due to the thermal conditions to which the holder 100 is subjected, each of the parts of the present holder 100 (the body 102, the cap 104 and the plug 106) is preferably formed from a similar material. This is to prevent the parts from expanding and contracting at different rates, and in different proportions from one another. It is also contemplated that different materials having similar thermal properties can also be used, where appropriate.

It will also be appreciated from the above-provided discussion that the spiral groove 144 must be formed or machined to within a fairly tight tolerance. In a present holder 100, a plug 106 having an overall length of about 1 inch is formed with a groove 144 having a cross-sectional area of 0.00031 inches². The groove 144 is formed using a 1/16 inch grooving tool having a rounded or curved profile, and cut to a depth of about 0.011 inches ±0.0005 inches (about 1/3 of a circle having a 1/16 inch diameter). The groove 144 has width of about 0.045 inches. Cross-sectional areas of up to about 0.001 inches can be used, however, the length of the plug 106 (and the spiral groove 144) is formed commensurately longer. For example, in a groove 144 having a cross-sectional area of 0.000553 inches², the groove 144 must be formed in a plug 106 having a length of about 2 inches (compared to a 1 inch plug 106 for the 0.00031 inches² area). It has been found that the cross-sectional area of the groove 144 is best formed at less than about 5.5 E-4 inches². The grooves 144 are formed in the plug 106 at a rate (density) of about 10 turns per linear inch of plug 106.

In use, the test strip 10 is placed in the holder 100 and the holder 100 is placed inside of the sterilizer chamber. The sterilizer chamber is evacuated and steam is then introduced into the device. Following a sterilization cycle, the specimen holder 100 is removed from the sterilizer chamber, and the test strip 10 is checked to verify whether air was adequately evacuated during the sterilization cycle as described before.

In the present disclosure, the words "a" or "an" are to be taken to include both the singular and the plural. Conversely, any reference to plural items shall, where appropriate, include the singular.

## Claims

1. An air removal test system for verifying adequate air removal during a sterilization cycle of a pre-vacuum type sterilizer, the system comprising:
a specimen holder including:
a tubular body defining an interior chamber;
a closure cap sealable on a first end of the tubular body; and
an end plug arranged in a second end of the tubular body, wherein the end plug includes a spiral formed channel providing a single ingress and egress flow path into and out of the interior chamber; and
a test strip including:
a base element formed from a thermally conductive material having a length and a width, the base element having a recess formed therein;
an acrylic adhesive layer provided on the base element;
a solid indicator chemical deposited in the recess;
a wicking material positioned at least in part in contact with the solid indicator chemical, the wicking material extending less than the length and width of the base element;
a film positioned over the base element, the wicking material and the solid indicator chemical; and
a coated paper disposed over the film, the coated paper including a window therein,, wherein when the specimen holder with the test strip in the interior chamber is placed in the pre-vacuum type sterilizer, the test strip indicates whether an adequate air removal has occurred during a sterilization cycle in which saturated steam is provided at 134°C for 3.5 minutes ± 5 seconds or at 121°C for 15 minutes ± 5 seconds, wherein the window in the coated paper is arranged at a predetermined distance from the solid indicator chemical such that the solid indicator chemical liquefies and wicks along the wicking material to change the color of the wicking material visible through the window to indicate that the adequate air removal has occurred during the sterilization cycle.

2. The air removal test system of claim 1, wherein the solid indicator chemical is salicylamide or ethoxy benzomide, and wherein the solid indicator chemical includes a dye.

3. The air removal test system of claim 2, wherein the dye is a blue dye present in a concentration of about 0.01 percent by weight of the indicator chemical.

4. The air removal test system of any of claims 1-3, wherein the film is a cast polypropylene.

5. The air removal test system of any of claim 1-4, wherein the base element is aluminum having a thickness of about 76 micrometers (3 mils)

6. The air removal test system of any of claims 1-5, wherein the coated paper has an adhesive backing.

7. The air removal test system of any of claims 1-6, wherein the coated paper has an acrylic coating thereon.

8. The air removal test system of claim 1, wherein the test strip has a length of about 100mm (4 inches) and the window has a length of about 25mm (1 inch), wherein the color change covers substantially the entire area of the wicking element visible through the window.

9. A method of verifying adequate air removal during a sterilization cycle of a prevacuum type, comprising:
introducing a test strip into a specimen holder including a tubular body defining an interior chamber, a closure cap sealable on a first end of the tubular body, and an end plug arranged in a second end of the tubular body, wherein the end plug provides a single ingress and egress flow path into and out of the interior chamber; wherein the test strip is configured to work in conjunction with the specimen holder to indicate whether an adequate air removal has occurred during a sterilization cycle in the prevacuum type sterilizer, the test strip having:
a base element formed from a thermally conductive material having a length and a width, the base element having a recess formed therein;
a solid indicator chemical deposited in the recess;
a wicking element positioned at least in part in contact with the solid indicator chemical, the wicking element extending less than the length and width of the base element;
a film layer positioned over the base element, the wicking element and the solid indicator chemical; and
a coated paper disposed over the film layer, the coated paper including a window therein, wherein the window is arranged away from the solid indicator chemical such that a portion of the wicking element is visible through the window;
placing the specimen holder including the test strip in the pre-vacuum type sterilizer;
running a pre-vacuum sterilization cycle at 134°C for 3.5 min ± 5 sec or 121°C for 15 min ± 5 sec; and
verifying an adequate air removal by inspecting a color change of the wicking element through the window, the color change of the wicking element being in response to adequate removal of air from the specimen holder,
wherein an adequate air removal is verified when the color change covers the entire area of the wicking element visible through the window.

## Patentansprüche

1. Luftentfernung-Testsystem zum Überprüfen einer adäquaten Luftentfernung im Zuge eines Sterilisationszyklus eines Sterilisators vom Typ Vorvakuumsterilisator, Folgendes beinhaltend:
einen Probenhalter, Folgendes umfassend:
einen röhrenförmigen Körper, welcher eine innere Kammer definiert;
eine Verschlusskappe, welche an einem ersten Ende des röhrenförmigen Körpers dicht verschlossen werden kann; und
einen Endstopfen, welcher in einem zweiten Ende des röhrenförmigen Körpers angeordnet ist, wobei der Endstopfen einen spiralförmigen Kanal umfasst, welcher einen einzigen Eintritts- und Austritts-Strömungsweg in die innere Kammer hinein und daraus heraus bereitstellt;
und einen Teststreifen, welcher Folgendes umfasst:
ein Basiselement, gebildet aus einem wärmeleitfähigen Material, welches eine Länge und eine Breite besitzt, wobei das Basiselement eine darin gebildete Vertiefung besitzt;
eine Acrylhaftschicht, welche an dem Basiselement bereitgestellt ist;
einen in der Vertiefung abgelagerten chemischen Indikatorfeststoff;
ein Dochtmaterial, welches mindestens teilweise in Kontakt mit dem chemischen Indikatorfeststoff positioniert ist, wobei das Dochtmaterial sich weniger weit als über die Länge und die Breite des Basiselements erstreckt;
einen Film, welcher über dem Basiselement, dem Dochtmaterial und dem chemischen Indikatorfeststoff positioniert ist; und
ein beschichtetes Papier, welches über dem Film angeordnet ist, wobei das beschichtete Papier ein Fenster darin enthält, wobei, wenn der Probenhalter mit dem Teststreifen in der inneren Kammer in dem Sterilisator vom Typ Vorvakuumsterilisator platziert ist, der Teststreifen anzeigt, ob eine adäquate Luftentfernung im Zuge eines Sterilisationszyklus eingetreten ist, bei welchem gesättigter Dampf über 3,5 Minuten ± 5 Sekunden bei 134 °C oder über 15 Minuten ± 5 Sekunden bei 121 °C bereitgestellt wird, wobei das Fenster in dem beschichteten Papier in einem vorbestimmten Abstand vom chemischen Indikatorfeststoff in einer Weise angeordnet ist, dass der chemische Indikatorfeststoff sich verflüssigt und entlang des Dochtmaterials migriert, um die Farbe des durch das Fenster sichtbaren Dochtmaterials zu ändern, um anzuzeigen, dass adäquate Luftentfernung im Zuge des Sterilisationszyklus eingetreten ist.

2. Luftentfernung-Testsystem nach Anspruch 1, bei welchem der chemische Indikatorfeststoff Salicylamid oder Ethoxybenzomid ist, und wobei der chemische Indikator Feststoff einen Farbstoff enthält.

3. Luftentfernung-Testsystem nach Anspruch 2, bei welchem der Farbstoff ein blauer Farbstoff ist, welcher in einer Konzentration von ungefähr 0,01 Gewichtsprozent der Indikatorchemikalie vorhanden ist.

4. Luftentfernung-Testsystem nach einem der Ansprüche 1 bis 3, bei welchem der Film ein gegossenes Polypropylen ist.

5. Luftentfernung-Testsystem nach einem der Ansprüche 1 bis 4, bei welchem das Basiselement Aluminium ist, welches eine Dicke von ungefähr 76 Mikrometern (3 Mils) besitzt.

6. Luftentfernung-Testsystem nach einem der Ansprüche 1 bis 5, bei welchem das beschichtete Papier eine selbstklebende Rückseite besitzt.

7. Luftentfernung-Testsystem nach einem der Ansprüche 1 bis 6, bei welchem das beschichtete Papier eine Acrylbeschichtung daran besitzt.

8. Luftentfernung-Testsystem nach Anspruch 1, bei welchem der Teststreifen eine Länge von ungefähr 100 mm (4 Zoll) und das Fenster eine Länge von ungefähr 25 mm (1 Zoll) aufweist, wobei die Farbänderung im Wesentlichen die gesamte Fläche des Dochtelements abdeckt, welche durch das Fenster sichtbar ist.

9. Verfahren zum Überprüfen einer adäquaten Luftentfernung im Zuge eines Sterilisationszyklus vom Typ Vorvakuum, Folgendes beinhaltend:
Einführen eines Teststreifens in einen Probenhalter, welcher einen röhrenförmigen Körper umfasst, welcher eine innere Kammer definiert, wobei eine Verschlusskappe an einem ersten Ende des röhrenförmigen Körpers dicht verschlossen werden kann, und ein Endstopfen in einem zweiten Ende des röhrenförmigen Körpers angeordnet ist, wobei der Endstopfen einen einzigen Eintritts- und Austritts-Strömungsweg in die innere Kammer hinein und daraus heraus bereitstellt; wobei der Teststreifen konfiguriert ist, um in Verbund mit dem Probenhalter zu arbeiten, um anzuzeigen, ob eine adäquate Luftentfernung im Zuge eines Sterilisationszyklus in dem Sterilisator vom Typ Vorvakuumsterilisator eingetreten ist, wobei der Teststreifen Folgendes aufweist:
ein Basiselement, gebildet aus einem wärmeleitfähigen Material, welches eine Länge und eine Breite besitzt, wobei das Basiselement eine hierin gebildete Vertiefung besitzt;
einen in der Vertiefung abgelagerten chemischen Indikatorfeststoff;
ein Dochtelement, welches mindestens teilweise in Kontakt mit dem chemischen Indikatorfeststoff positioniert ist, wobei das Dochtelement sich weniger weit als über die Länge und die Breite des Basiselements erstreckt;
eine Filmschicht, welche über dem Basiselement, dem Dochtelement und dem chemischen Indikatorfeststoff positioniert ist; und
ein beschichtetes Papier, welches über dem Film angeordnet ist, wobei das beschichtete Papier ein Fenster darin enthält, wobei das Fenster vom chemischen Indikatorfeststoff in einer Weise entfernt angeordnet ist, dass ein Abschnitt des Dochtelements durch das Fenster sichtbar ist;
Platzieren des Probenhalters mitsamt dem Teststreifen in dem Sterilisator vom Typ orvorvakuumsterilisator;
Durchführen eines Vorvakuumsterilisationszyklus über 3,5 Min. ± 5 Sek. bei 134 °C oder über 15 Min. ± 5 Sek. bei 121 °C; und
Überprüfen einer adäquaten Luftentfernung durch Inspizieren einer Farbänderung des Dochtelementes durch das Fenster, wobei eine Farbänderung des Dochtelementes in Reaktion auf adäquate Luftentfernung aus dem Probenhalter erfolgt,
wobei eine adäquate Luftentfernung verifiziert ist, wenn die Farbänderung die gesamte Fläche des Dochtelementes bedeckt, welche durch das Fenster sichtbar ist.

## Revendications

1. Système de test pour éliminer l'air permettant de vérifier l'élimination adéquate de l'air au cours d'un cycle de stérilisation d'un stérilisateur de type à prévide, le système comprenant :
un porte-échantillons incluant :
un corps tubulaire définissant un compartiment intérieur ;
un capuchon de fermeture apte à être scellé sur une première extrémité du corps tubulaire ; et
un bouchon d'extrémité agencé dans une seconde extrémité du corps tubulaire, dans lequel le bouchon d'extrémité inclut un canal en forme de spirale fournissant une trajectoire d'écoulement à entrée et sortie unique dans le compartiment intérieur et hors de celui-ci ; et
une bandelette de test incluant :
un élément de base formé à partir d'un matériau thermiquement conducteur présentant une longueur et une largeur, l'élément de base présentant un évidement formé à l'intérieur de celui-ci ;
une couche adhésive en acrylique prévue sur l'élément de base ;
un produit chimique indicateur solide déposé dans l'évidement ;
un matériau drainant positionné au moins en partie en contact avec le produit chimique indicateur solide, le matériau drainant s'étendant sur moins que la longueur et la largeur de l'élément de base ;
un film positionné sur l'élément de base, l'élément drainant et le produit chimique indicateur solide ; et
un papier revêtu disposé sur le film, le papier revêtu incluant une fenêtre à l'intérieur de celui-ci, dans lequel, lorsque le porte-échantillons avec la bandelette de test dans le compartiment intérieur est placé dans le stérilisateur de type à prévide, la bandelette de test indique si une élimination adéquate de l'air s'est produite au cours d'un cycle de stérilisation dans lequel de la vapeur saturée est fournie à 134°C pendant 3,5 minutes ± 5 secondes ou à 121°C pendant 15 minutes ± 5 secondes, dans lequel la fenêtre dans le papier revêtu est agencée à une distance prédéterminée par rapport au produit chimique indicateur solide, de telle sorte que le produit chimique indicateur solide se liquéfie et draine le long du matériau drainant pour changer la couleur du matériau drainant visible à travers la fenêtre pour indiquer que l'élimination adéquate de l'air s'est produite au cours du cycle de stérilisation.

2. Système de test d'élimination de l'air selon la revendication 1, dans lequel le produit chimique indicateur solide est le salicylamide ou l'éthoxy-benzomide, et dans lequel le produit chimique indicateur solide inclut un colorant.

3. Système de test d'élimination de l'air selon la revendication 2, dans lequel le colorant est un colorant bleu présent en une concentration d'environ 0,01 pour cent en poids du produit chimique indicateur.

4. Système de test d'élimination de l'air selon l'une quelconque des revendications 1 à 3, dans lequel le film est un polypropylène coulé.

5. Système de test d'élimination de l'air selon l'une quelconque des revendications 1 à 4, dans lequel l'élément de base est de l'aluminium présentant une épaisseur d'environ 76 micromètres (3 mils).

6. Système de test d'élimination de l'air selon l'une quelconque des revendications 1 à 5, dans lequel le papier revêtu présente un support adhésif.

7. Système de test d'élimination de l'air selon l'une quelconque des revendications 1 à 6, dans lequel le papier revêtu présente un revêtement en acrylique sur celui-ci.

8. Système de test d'élimination de l'air selon la revendication 1, dans lequel la bandelette de test présente une longueur d'environ 100 mm (4 pouces) et la fenêtre présente une longueur d'environ 25 mm (1 pouce), dans lequel le changement de couleur recouvre sensiblement toute la zone de l'élément drainant visible à travers la fenêtre.

9. Procédé de vérification de l'élimination adéquate de l'air au cours d'un cycle de stérilisation d'un type à prévide, comprenant :
l'introduction d'une bandelette de test dans un porte-échantillons incluant un corps tubulaire définissant un compartiment intérieur, un capuchon de fermeture apte à être scellé sur une première extrémité du corps tubulaire, et un bouchon d'extrémité agencé dans une seconde extrémité du corps tubulaire, dans lequel le bouchon d'extrémité inclut une trajectoire d'écoulement à entrée et sortie unique dans le compartiment intérieur et hors de celui-ci ; dans lequel la bandelette de test est configurée pour travailler en relation avec le porte-échantillons pour indiquer si une élimination adéquate de l'air s'est produite au cours d'un cycle de stérilisation dans le stérilisateur de type à prévide, la bandelette de test présentant :
un élément de base formé à partir d'un matériau thermiquement conducteur présentant une longueur et une largeur, l'élément de base présentant un évidement formé dans celui-ci ;
un produit chimique indicateur solide déposé dans l'évidement ;
un élément drainant positionné au moins en partie en contact avec le produit chimique indicateur solide, l'élément drainant s'étendant sur moins que la longueur et la largeur de l'élément de base ;
une couche de film positionnée sur l'élément de base, l'élément drainant et le produit chimique indicateur solide ; et
un papier revêtu disposé sur la couche de film, le papier revêtu incluant une fenêtre à l'intérieur de celui-ci, dans lequel la fenêtre est agencée à distance du produit chimique indicateur solide, de telle sorte qu'une partie de l'élément drainant est visible à travers la fenêtre ;
la mise en place du porte-échantillons incluant la bandelette de test dans le stérilisateur de type à prévide ;
la réalisation d'un cycle de stérilisation à prévide à 134°C pendant 3,5 minutes ± 5 secondes ou à 121°C pendant 15 minutes ± 5 secondes ; et
la vérification de l'élimination adéquate de l'air en examinant un changement de couleur de l'élément drainant à travers la fenêtre, le changement de couleur de l'élément drainant étant en réponse à l'élimination adéquate de l'air hors du porte-échantillons,
dans lequel une élimination adéquate de l'air est vérifiée, lorsque le changement de couleur recouvre toute la zone de l'élément drainant visible à travers la fenêtre.
